# EUROPÄISCHE PATENTSCHRIFT

(11) **EP 1 229 867 B1**
(45) Veröffentlichungstag und Bekanntmachung des Hinweises auf die Patenterteilung: **02.11.2006**
(21) Anmeldenummer: 00979600.4
(22) Anmeldetag: 17.11.2000
(51) Int. Cl.: A61F 2/14, A61K 9/00, A61K 47/48, A61K 41/00

(54) **OPHTHALMOLOGISCHES IMPLANTAT**
OPHTHALMOLOGIC IMPLANT
IMPLANT OPHTALMOLOGIQUE

(30) Priorität: 19.11.1999 DE 19955836
(43) Veröffentlichungstag der Anmeldung: 14.08.2002
(73) Patentinhaber: HAMPP, Norbert, 35287 Amöneburg-Rossdorf (DE)
(72) Erfinder: HEITZ, Walter, 35274 Kirchhain (DE); GREINER, Andreas, 35043 Marburg (DE); HESSE, Lutz, 35039 Marburg (DE); HAMPP, Norbert, 35287 Amöneburg-Rossdorf (DE)
(74) Vertreter: Rau, Albrecht
(86) Internationale Anmeldenummer: PCT/EP2000/011477
(87) Internationale Veröffentlichungsnummer: WO 2001/035867

(56) Entgegenhaltungen:
- EP-A- 0 791 361
- WO-A-96/23543
- DE-A- 19 700 082
- US-A- 5 618 553
- BRUIN P ET AL: "AUTOCLAVABLE HIGHLY CROSS-LINKED POLYURETHANE NETWORKS IN OPHTHALMOLOGY" BIOMATERIALS,GB,ELSEVIER SCIENCE PUBLISHERS BV., BARKING, Bd. 14, Nr. 14, 1. November 1993 (1993-11-01), Seiten 1089-1097, XP000412515 ISSN: 0142-9612
- CHEMICAL ABSTRACTS, vol. 133, no. 23, 4. Dezember 2000 (2000-12-04) Columbus, Ohio, US; abstract no. 325672, KOJIMA, MASANOBU ET AL: "Lenses containing photocatalysts for eyes" XP002169764 & JP 2000 296174 A (NIDEK K. K., JAPAN) 24. Oktober 2000 (2000-10-24)

## Beschreibung

Die Erfindung betrifft ein ophthalmologisches Implantat aus einem polymeren Werkstoff, welches bevorzugt selbst als Intraokularlinse ausgebildet ist oder als Zusatzausrüstung für Intraokularlinsen eingesetzt wird. Das Implantat dient insbesondere zur Prophylaxe oder/und zur Behandlung von Kapseltrübung nach der Implantation einer synthetischen Linse und nach Abschluss der Wundheilung. Die Behandlung kann bei Bedarf wiederholt werden.

Der medizinische Fachbegriff für eine eingetrübte Linse ist die Katarakt. Tritt eine Katarakt bei der natürlichen Linse auf, wird diese als Primäre Katarakt bezeichnet. Tritt eine Trübung nach Explantation der natürlichen Linse und Implantation einer synthetischen Linse auf, wird dies als Sekundäre Katarakt oder als "cataracta secondaria" (Nachstar) bezeichnet. Häufig verwendet wird in der Fachsprache der englische Begriff "posterior capsule opacification" oder die Abkürzung PCO.

Eine Katarakt wird im Allgemeinen durch die chirurgische Entfernung der eingetrübten Linse und Implantation einer allogenen Intraokularlinse in den verbliebenen Linsenkapselsack behandelt. Dieser Eingriff gehört zu den häufigsten ophthalmologischen Operationen. Allerdings gelingt es meist nicht, den Kapselsack vollständig von verbliebenen Linsenepithelzellen zu säubern. Häufig verbleiben Zellen am Äquator des Linsensackes, proliferieren und migrieren von dort zentral, was später zu einer Trübung im Bereich des Kapselsackes führt, d.h. der Ausbildung einer Sekundären Katarakt. Dies erfordert einen chirurgischen Eingriff, der Kosten verursacht und für den Patienten belastend ist und Risiken beinhaltet (z.B. Ausbildung einer Netzhautablösung).

Ein besonderes Problem stellt die Nachstarentwicklung bei Kindern und jungen Erwachsenen dar. Hier ist die Regenerationsrate der Linsenepithelzellen so ausgeprägt, dass es teilweise bereits nach wenigen Wochen zu einer erneuten starken Eintrübung kommt. Die Entwicklung eines Nachstars behindert nicht nur das Sehen, sondern erfordert unter Umständen weitere operative und diagnostische Maßnahmen, wie etwa eine Laserkoagulation oder detaillierte Untersuchungen der Netzhaut.

Die Zahl der implantierten Intraokularlinsen hat in den vergangenen Jahren stetig zugenommen. Derzeit werden schätzungsweise 400.000 Intraokularlinsen pro Jahr in Deutschland im Rahmen einer Kataraktoperation implantiert. Nach komplikationsfreier Operation und unauffälligem postoperativem Heilverlauf ist bei einer erheblichen Zahl von Patienten mit einer erneuten Visusminderung durch einen Nachstar zu rechnen, der durch die Proliferation verbliebener Linsenepithelzellen verursacht wird.

Um diese Proliferationen einzudämmen, wurden bisher verschiedene Verfahren angewandt.

US 5,576,345 beschreibt die Verwendung von Taxol bzw. Taxolderivaten zur Verhinderung von sekundären Katarakten. Dazu wird gemäß US 5,576,345 Taxol oder ein Taxolderivat in den intraokularen Bereich verabreicht, wobei die Intraokularlinse als Träger für den Wirkstoff dienen kann. Das Erzielen einer Depotwirkung durch Verwendung einer immobilisierten, photochemisch aktivierbaren Komponente wird nicht in Betracht gezogen.

WO 99/52570 beschreibt eine beschichtete Intraokularlinse umfassend ein Substratmaterial und eine Beschichtung, welche des Risiko einer Trübung nach Implantation der synthetischen Linse verringern soll. Als Beschichtungsmaterial werden dabei hydrophobe Arylacrylmonomere eingesetzt.

WO 97/06838 betrifft eine intraokulare Linse, auf die ein Trägermedium und darin eingebettet Wirkstoffe aufgebracht sind.

Eine weitere Möglichkeit besteht in der vollständigen Entfernung von Linsenepithelzellen aus dem Kapselsack während der Operation (R.C. Humphry, E.G. Davies, T.J.C. Jakob, G.M. Thompson: The human anterior lens capsule - an attempted chemical debridement of epithelial cells by ethylenediaminetetracetic acid (EDTA) and trypsin. Br. J. Ophthalmol. 72 (1988) 406-408; O. Nishi, K. Nishi, M. Hikida: Removal of lens epithelial cells by dispersion with enzymatic treatment followed by aspiration. Ophthalmic. Surg. 22 (1991) 444-450).

Bei der Entfernung von Linsenepithelzellen aus dem Kapselsack während der Operation kann chirurgisch jedoch nicht sichergestellt werden, dass alle Epithelzellen vollständig entfernt worden sind. Dies ist auch nicht durch einen zusätzlichen Einsatz von chemischen Wirkstoffen zu erreichen. Die Anwendung aggressiver chemischer Reagenzien verbietet sich aus medizinischer Sicht, weil eine Schädigung des Kapselsackes oder benachbarter Gewebe nicht auszuschließen wäre.

Weiterhin wurde versucht, die Proliferation von Linsenepithelzellen durch eine Modifizierung der Intraokularlinsen-Oberfläche bzw. alternative Intraokularlinsen-Materialien zu hemmen (G. Duncan, I.M. Wormstone, C.S.C. Liu, J.M. Marcantonia, P.D. Davies: Thapsigargin-coated intraocular lenses inhibit human lens cell growth. Nature 3 (1997) 1026-1028; R.J. Siezen, C.M. Coppin, G.B. Benedek: Process for preventing or reversing cataract formation using protein modification reagents. US 4,665,089 (1987); L. Hesse, L. Freisberg, H. Bienert, H. Richter, C.

Kreiner, C. Mittermayer: Verminderung des Nachstars durch Plasmaätzung von Intraokularlinsen. Ophthalmologe 94 (1997) 821-825).

Intraokularlinsen mit einer modifizierten Oberfläche oder aus modifizierten Materialien, die dauerhaft einen Bewuchs mit Epithelzellen verhindern sollen, werden derzeit erprobt. Die Bildung eines Nachstars kann dadurch allein allerdings nicht ausgeschlossen werden. Die Linsenepithelzellen können auch auf der Innenseite des Kapselsackes wachsen und dann zentral migrieren. Zudem bewirken dauerhaft epitheltoxische Maßnahmen, dass die Wundheilung nach der Implantation einer synthetischen Linse beeinträchtigt wird.

Ein weiteres Verfahren umfasst den Einsatz von proliferationshemmenden oder cytotoxischen Substanzen in den Kapselsack (D.W. Cash: Method of inhibiting cataracts by topical application of a 2-substituted 1,2-benzisoselenazol-3(2H)-one. US 4,778,815 (1988); J.M. Emery, R.Y. Chan: Mitotic inhibitors preventing posterior lens capsule opacification. US 4,657,930 (1987); J.M. Ruiz, A. Medrano, J.L. Alio: Inhibition of posterior capsule opacification by 5-fluorouracil in rabbits. Ophthalmic. Res. 22 (1990) 201-208).

Der Einsatz von proliferationshemmenden oder cytotoxischen Substanzen oder verwandten Pharmaka in den Kapselsack behindert jedoch die Wundheilung nach einer Operation.

Die Häufigkeitsangaben zum Nachstar variieren und sind wesentlich vom Nachbeobachtungszeitraum abhängig. Trotz den genannten Maßnahmen, wie etwa veränderte chirurgische Techniken und modifiziertes Linsendesign beträgt die Inzidenz des Nachstars 20-50 % innerhalb eines Zeitintervalles von 2-5 Jahren (D.J. Apple, K.D. Solomon, M.R. Tetz, El. Assia, E.Y. Holland, V.F.C. Legler, J.C. Tsai: Posterior capsule opacification. Surv. Ophthalmol. 37 (1992) 73-116; C. Ohadi, H. Moreia, P. McDonell: Posterior capsule opacification. Curr. Opin. Ophthalmol. 2 (1991) 46-52).

Dies zeigt, dass die Nachstarbildung derzeit noch nicht effektiv verhindert werden kann.

Alle bekannten Verfahren haben zudem den Nachteil, dass im Falle einer Nachstarbildung keine Möglichkeit zu einer Wiederholung der therapeutischen Maßnahme oder zu einer nochmaligen intensivierten Anwendung besteht, ohne dass dazu das Auge operativ eröffnet werden müsste.

Die Proliferation von Linsenepithelzellen ist ein komplizierter Prozess, dessen genauer Mechanismus gegenwärtig nicht bekannt ist. Neben einem Verlust der Kontaktinhibition beeinflussen Entzündungsmediatoren und andere Faktoren die Entstehung des Nachstars. Eine medikamentöse Behandlung des Nachstars wird allein dadurch behindert, dass nur wenige Substanzen nach Auftropfen auf die Hornhaut in die Vorderkammer aufgenommen werden. Weiterhin erschwert die Intraokularlinse den Austausch von Kammerwasser im Kapselsack. Pharmakologisch ist das Kammerwasservolumen als ein eigenständiges Kompartiment zu betrachten.

Dieser Umstand erfordert, dass die Applikation einer hemmenden Substanz in die Vorderkammer während oder am Ende der Operation zur Linsenimplantation erfolgen muss. Die in Frage kommenden Wirkstoffe weisen aber alle einen unerwünschten hemmenden Effekt auf die postoperative Wundheilung auf.

Der Artikel **BRUIN P ET AL: 'AUTOCLAVABLE HIGHLY CROSS-LINKED POLYURETHANE NETWORKS IN OPHTHALMOLOGY' BIOMATERIALS,GB,ELSEVIER SCIENCE PUBLISHERS BV., BARKING, Bd. 14, Nr. 14, 1. November 1993 (1993-11-01), Seiten 1089-1097, XP000412515 ISSN: 0142-9612,** gegen den der **Anspruch 1** abgegrenzt ist, beschreibt ein ophthalmologisches Implantat, welches aus einem polymeren Werkstoff besteht und eine chemische Komponente wie beispielsweise Cumarin zur Absorption schädlicher UV-Strahlen umfasst.

Eine Aufgabe der Erfindung bestand daher darin, ein ophthalmologisches Implantat, wie etwa eine Intraokularlinse oder eine Zusatzausrüstung für bereits verfügbare Intraokularlinsen bereitzustellen, mit dem die oben beschriebenen Komplikationen nach einer Kataraktoperation vermieden und die Proliferation von Linsenepithelzellen nachhaltig unterdrückt werden kann.

Diese Aufgabe wird erfindungsgemäß gelöst durch ein ophthalmologisches Implantat zur Prophylaxe oder/und zur Behandlung von Kapseltrübung nach der Implantation einer synthetischen Linse, welches dadurch gekennzeichnet ist, dass es aus einem polymeren Werkstoff besteht und zumindest bereichsweise eine chemische Komponente in immobilisierter Form umfasst, aus der durch photochemische Aktivierung ein pharmazeutischer Wirkstoff freisetzbar ist. Der Wirkstoff weist bevorzugt eine zelltoxische Wirkung auf. Besonders bevorzugt weist der freigesetzte Wirkstoff eine epitheltoxische, insbesondere eine Linsenepithel-toxische Wirkung auf.

Durch das erfindungsgemäße Implantat ist es möglich, die gewünschte Arzneimittelwirkung, z.B. eine zelltoxische, insbesondere epitheltoxische Wirkung nicht unmittelbar nach der Operation, sondern erst zu einem späteren Zeitpunkt, z.B. frühestens nach Abschluss der Wundheilung, bei Bedarf zu aktivieren und ggf. mehrere Jahre nach der Implantation erneut zu reaktivieren. Erfindungsgemäß ist der Wirkstoff, z.B. ein epitheltoxischer Wirkstoff nicht dauerhaft und damit nicht schon unmittelbar nach der Implantation aktiv, was die Wundheilung beeinträchtigen würde. Vielmehr enthält das ophthalmologische Implantat den pharmazeutischen Wirkstoff in einer inaktiven Form. Der Wirkstoff kann somit in einer pharmakologisch inaktiven Form bei der Kataraktoperation durch Implantation eines medikamentösen Depots appliziert werden. Das Implantat umfasst also ein photoaktivierbares Wirkstoffdepot. Die Wirkstofffreisetzung kann dann nach dem Abschluss der Wundheilung nichtinvasiv durch photochemische Aktivierung induziert werden. Durch die Aktivierung wird der vorher fixierte Wirkstoff in geeigneten Mengen in das geringe Kammerwasservolumen freigesetzt, um dort therapeutisch relevante Konzentrationen einzustellen, etwa um gebildete Linsenepithelzellen abzutöten und das Wachstum neuer Linsenephitefzellen zu verhindern.

Von besonderem Vorteil ist dabei die Möglichkeit, den therapeutischen Effekt durch eine zeitlich versetzte, mehrmalige nicht-invasive Wirkstofffreisetzung steuern und verbessern zu können.

Ein weiterer Vorteil besteht darin, dass diese Behandlung in allen augenheilkundlichen Einrichtungen mit Laserausstattung ambulant erfolgen kann und damit der bisher notwendige (Laser-)chirurgische Eingriff mit Zerstörung der Hinterkapsel entfällt.

Der Wirkstoff kann bereits im Anfangsstadium einer Kapseltrübung, z.B. beim ersten Auftreten eines Zuwachsens der implantierten Linse mit Epithelzellen, was z.B. mit Hilfe eines Mikroskops festgestellt wurde, gezielt freigesetzt werden. Dadurch ist eine Behandlung der Kapseltrübung im Anfangsstadium, also noch bevor eine erhebliche Visusbehinderung der betroffenen Person eintritt, möglich.

Das erfindungsgemäße Implantat wird bevorzugt zur Behandlung von Kapseltrübung eingesetzt. Es ist aber auch möglich, das erfindungsgemäße Implantat zur Prophylaxe als vorbeugende Maßnahme einzusetzen, indem zumindest ein Teil des Wirkstoffs nach der Wundheilung, aber vor dem Auftreten einer Kapseltrübung, freigesetzt wird, um z.B. bei der Operation nicht entfernte, restliche Zellen, insbesondere Linsenepithelzellen, abzutöten.

Bei dem erfindungsgemäßen ophthalmologischen Implantat handelt es sich bevorzugt um eine Intraokularlinse oder um eine Zusatzausrüstung für eine Intraokularlinse. Zur Vermeidung oder Behandlung von Kapseltrübung nach der Implantation ist das Implantat aus einem polymeren Werkstoff hergestellt, der ganz oder zonenweise eine chemische Komponente in immobilisierter Form enthält, aus der durch photochemische Aktivierung ein pharmazeutischer Wirkstoff freigesetzt wird, der bevorzugt eine zelltoxische, bevorzugt eine epitheltoxische Wirkung aufweist und insbesondere die Proliferation von Linsenepithelzellen hemmt, unterbindet oder vorhandene Linsenephithelzellen abtötet.

In einer ersten Ausführungsform stellt das ophthalmologische Implantat eine Intraokularlinse ohne notwendige Zusatzausrüstung dar. Die erfindungsgemäßen Intraokularlinsen sind entweder vollständig oder nur teilweise, z.B. nur einzelne Zonen, wie etwa eine toroidfömige Randzone, aus dem erfindungsgemäßen polymeren Material hergestellt. Eine weitere Ausführungsform ist, dass eine aus nicht mit Wirkstoff beladenem Polymer hergestellte Intraokularlinse mit einer dünnen Schicht des erfindungsgemäß mit Wirkstoff beladenen Polymers überzogen ist.

Eine weitere Ausführungsform sind Zusatzausrüstungen zu konventionellen Intraokularlinsen. Die Zusatzausrüstung besteht vorteilhaft aus einem separaten Element, das selbst nicht die Funktion einer Linse hat, aber zusammen mit einer konventionellen Intraokularlinse implantiert wird, z.B. ein toroidförmiges polymeres Koimplantat.

Grundsätzlich kann das erfindungsgemäße ophthalmologische Implantat eine chemische Komponente umfassen, aus der durch photochemische Aktivierung ein beliebiger, für das Auge vorteilhafter pharmazeutischer Wirkstoff freisetzbar ist. Bevorzugt ist aus der chemischen Komponente in immobilisierter Form ein pharmazeutischer Wirkstoff freisetzbar, der eine zelltoxische, insbesondere eine epitheltoxische Wirkung aufweist. Weitere Wirkstoffe, die in einer inaktiven Form immobilisiert sein können, umfassen beispielsweise Antibiotika, wie etwa antimikrobielle Substanzen, die bevorzugt gegen übliche im Auge auftretende Pathogene wirksam sind, entzündungshemmende Materialien, wie etwa Kortikosteroide und nicht-steroidale entzündungshemmende Mittel sowie antivirale (z.B. Ganciclovir) oder fungizide Wirkstoffe. Weiterhin ist es möglich, Hormon- oder Enzymwirkstoffe zu immobilisieren, wie etwa den Anti-Fibroplastenwachstumsfaktor oder Wirkstoffe, welche eine proliferative Vitreoretinopathie oder eine Gewebefibrose hemmen. Weitere geeignete Wirkstoffe umfassen beispielsweise Antikörper, z.B. gegen den transformierenden Wachstumsfaktor-β, und Inhibitoren, z.B. von Peptidpromotoren, Inhibitoren von Wachstumsfaktoren und Kinaseinhibitoren. Das erfindungsgemäße ophthalmologische Implantat kann einen oder beliebige Gemische von mehreren der oben genannten Wirkstoffe in immobilisierter Form enthalten.

Der Wirkstoff kann an der Oberfläche oder/und im Inneren des polymeren Werkstoffs angeordnet sein. Gemäß einer bevorzugten Ausführung ist der Wirkstoff an den polymeren Werkstoff oder eine polymere Komponente davon über photochemisch spaltbare Linkermoleküle kovalent gekoppelt. Die Linkermoleküle werden bevorzugt aus der Gruppe der Zimtsäuren, Cumarine oder Derivaten davon ausgewählt.

Eine alternative Ausführungsform besteht darin, dass der Wirkstoff in Form von photospaltbaren Oligomeren oder Polymeren in den polymeren Werkstoff der Intraokularlinse oder Zusatzausrüstung eingebracht ist, wobei die Fixierung z.B. durch einen mechanischen Einschluss oder/und durch kovalente Quervernetzung erfolgt.

Eine weitere Alternative für die Einbindung des Wirkstoffs besteht darin, dass makromolekulare Konjugate aus dem Wirkstoff und einem oder mehreren Hilfsstoffen eingebracht sind, wobei die Hilfsstoffe der Quervernetzung des Wirkstoffes dienen und die Löslichkeit des makromolekularen Konjugates im polymeren Werkstoff verbessern und photospaltbare Gruppen enthalten.

Eine weitere alternative Lösung besteht darin, dass ein photoaktivierbarer Vorläuferwirkstoff dem polymeren Werkstoff in nanodisperser Form zugemischt ist, wobei die mittlere Teilchengröße < 1 µm, bevorzugt < 500 nm, beträgt.

Die immobilisierte chemische Komponente ist selbst pharmazeutisch inaktiv. Bevorzugt handelt es sich bei der chemischen Komponente um eine an das Implantat immobilisierte Form des Wirkstoffes. Da der Wirkstoff seine gewünschte Wirkung, z.B. eine epitheltoxische Wirkung aber nur in direktem Kontakt mit den Epithelzellen entfalten kann, wird er durch die Immobilisierung inaktiviert.

In einer weiteren bevorzugten Ausführungsform liegt eine inaktive Wirkstoff-Vorstufe in Form eines Konjugats, das an andere Moleküle gebundenen Wirkstoff enthält, oder/und in Form eines Polymers oder Oligomers von mehreren Wirkstoffmolekülen vor, wobei die aktive Wirkstoffform dann durch Freisetzung einzelner Wirkstoffmoleküle erhalten wird.

Erfindungsgemäß kann der Wirkstoff zu einem beliebigen Zeitpunkt gezielt durch photochemische Freisetzung aktiviert werden. Dadurch ist es möglich, den Wirkstoff in einer inaktiven Form als Depot einzubringen und erst zu einem gewünschten späteren Zeitpunkt zu aktivieren. So kann beispielsweise zunächst abgewartet werden, bis die Wundheilung nach der Operation abgeschlossen ist, bevor eine Wirkung, z.B. eine epitheltoxische Wirkung aktiviert wird. Weiterhin ist es möglich, den Wirkstoff gezielt bei Auftreten von Kapseltrübung auch noch Jahre nach der Operation zu aktivieren. Die photochemische Aktivierung wird bevorzugt durch Bestrahlen des Vorläuferwirkstoffs mit Licht ausgeführt. Je nach Art des Vorläuferwirkstoffes, beispielsweise ob es sich um einen mit einem photochemisch spaltbaren Linker immobilisierten Wirkstoff oder um ein Wirkstoffpolymer handelt, kann der Wellenlängenbereich des einzustrahlenden Lichts ausgewählt und vorbestimmt werden. Beispielsweise kann durch die Verwendung von spezifischen Linkergruppen, die nur mit Licht ganz bestimmter Wellenlänge gespalten werden, eine hoch selektive Freisetzung des Wirkstoffs erhalten werden. Bevorzugt werden Vorläuferwirkstoffe verwendet, die durch Einstrahlung von UV-Licht (100 - 400 nm), sichtbarem Licht (400 - 750 nm) oder Infrarotlicht (750 nm - 1000 µm) gespalten werden können.

In therapeutischer Hinsicht ist es vorteilhaft, wenn der Wirkstoff durch eingestrahltes Licht in einem eng begrenzten Spektralbereich freisetzbar ist, der nicht mehr als 50%, bevorzugt nicht mehr als 25% des sichtbaren Spektralbereichs einnimmt. So kann durch Tragen geeigneter Augengläser, die den relevanten Spektralbereich ausfiltern, eine vorzeitige Freisetzung durch Tageslicht während der Wundheilung vermieden werden.

Besonders günstig ist es, wenn die photochemische Freisetzung des Wirkstoffs durch eine Zweiphotonenabsorption von Licht mit zwei gleichen oder zwei verschiedenen Wellenlängen induzierbar ist. Dabei liegt die für die photochemische Freisetzung notwendige Energie vorteilhafterweise in einem Wellenlängebereich unterhalb des sichtbaren Spektralbereichs im sogenannten ultravioletten Bereich. Da die Transmission der natürlichen Hornhaut im UV-Bereich sehr gering ist, kann auf das Tragen spezieller Augengläser (siehe oben), die wegen der notwendigen spektralen Eigenschaften farbig sind und deshalb kosmetisch unerwünscht sein könnten, verzichtet werden.

Erfindungsgemäß bevorzugt wird für die photochemische Aktivierung eine relativ hohe Energie benötigt, so dass es nicht zu einer unbeabsichtigten vorzeitigen Freisetzung des Wirkstoffes ohne Anwendung der vorgesehenen photochemischen Aktivierung kommen kann. Insbesondere sollte eine vorzeitige Freisetzung durch Wärme vermieden werden.

Die Erfindung umfasst weiterhin ein Verfahren zur Herstellung eines ophthalmologischen Implantats umfassend die Schritte:
- Bereitstellen eines polymeren Werkstoffes,
- Immobilisieren einer chemischen Komponente auf oder/und in den polymeren Werkstoff, aus der durch photochemische Aktivierung ein pharmazeutischer Wirkstoff freisetzbar ist, der bevorzugt eine zelltoxische, insbesondere eine epitheltoxische Wirkung aufweist, und
- Bilden eines ophthalmologischen Implantats aus dem polymeren Werkstoff.

Das Herstellungsverfahren umfasst zwei bevorzugte Varianten. So kann das Implantat aus einem polymeren Werkstoff gebildet werden, der die chemische Komponente bereits enthält, oder es kann zunächst das Implantat aus einem nicht-dotierten Werkstoff gebildet werden, und die chemische Komponente wird dann erst an das Implantat immobilisiert. Diese Vorgehensweise ermöglicht z.B. das Dotieren von herkömmlichen Augenimplantaten mit chemischen Komponenten, wodurch erfindungsgemäße Implantate erhalten werden können.

Weiterhin umfasst die Erfindung die Verwendung des ophthalmologischen Implantats zur Prophylaxe oder zur Behandlung von Kapseltrübung nach der Implantation einer synthetischen Linse.

Im Folgenden wird die Erfindung anhand von Ausführungsbeispielen und Zeichnungen näher erläutert. Es zeigen:
- Fig. 1: Intraokularlinse 1 und Zusatzausrüstung 2 für eine Intraokularlinse in Gestalt eines Toroids;
- Fig. 2: schematische Darstellung der photochemisch induzierten Freisetzung eines an ein polymeres Gerüst 3 mittels photospaltbarer Linkermoleküle 4 gebundenen Wirkstoffs 5;
- Fig. 3: ein typisches Absorptionspektrum der Hornhaut des Auges mit hoher Transmission im sichtbaren und nahen infraroten Spektralbereich (400 - 1200 nm) und geringer Transmission im nahen UV-Bereich (< 400 nm);
- Fig. 4: schematische Darstellung des Auges 6 mit implantierter polymerer Intraokularlinse 7, natürlicher Hornhaut 8 und der Lichtführung für eine Zweiphotonenabsorption zur Freisetzung des Wirkstoffs aus einer erfindungsgemäßen Intraokularlinse mittels zweier Lichtstrahlen 9a und 9b, deren Wellenlängen im Bereich hoher Transmission der Hornhaut liegen und die sich im Bereich der implantierten Intraokularlinse überlappen.

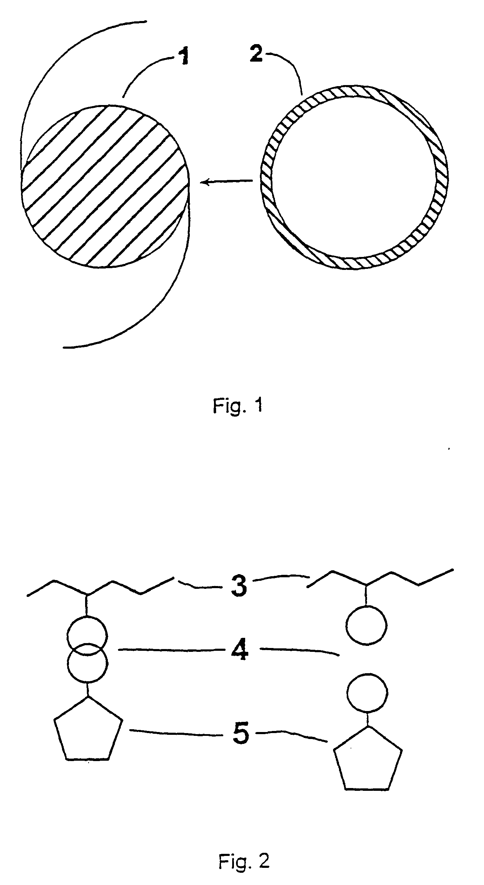

Im Folgenden werden einige bevorzugte Ausführungsformen der Erfindung sowie zur Durchführung der Erfindung geeignete Materialien erläutert.
1. Intraokularlinse und Zusatzausrüstung dafür
   Die Intraokularlinse 1 kann komplett aus dem Wirkstoff-beladenen Polymer hergestellt werden. Alternativ ist es aber auch möglich, nur eine Zone von einigen Mikrometern Dicke mit dem Wirkstoffbeladenen Polymer zu versehen, z.B. durch Belacken einer Intraokularlinse. Aus dieser Oberflächenschicht kann der Wirkstoff besonders rasch freigesetzt werden, da nur kurze Diffusionswege notwendig sind.
   Die Zusatzausrüstung 2 gemäß Fig. 1 besteht beispielsweise aus einem ringförmigen Körper, dessen Innenradius mit dem Außenradius der Intraokularlinse 1 übereinstimmt. Der ringförmige Körper, der den epitheltoxischen Wirkstoff enthält, wird ggf. vor der Implantation durch Kleben mit der Intraokularlinse verbunden oder ohne mechanische Verbindung koimplantiert. So können konventionelle Intraokularlinsen mit einem erfindungsgemäßen Wirkstoffschutz bei der Implantation versehen werden. Der Vorteil dieses Verfahrens ist, dass in der Intraokularlinse keine strukturellen Änderungen herbeigeführt werden müssen, die potentiell die Langzeitstabilität der Intraokularlinse vermindern würden, insbesondere dann, wenn große Mengen an Wirkstoff freigesetzt werden müssen. Ein weiterer Vorteil ist, dass eine Trübung des Werkstoffs der Zusatzausrüstung oder gewollte Färbung der Zusatzausrüstung die visuelle Wahrnehmung nicht beeinträchtigt, da die Zusatzausrüstung bevorzugt peripher im Kapselsack positioniert wird.
2. Polymere Werkstoffe für Intraokularlinsen mit kovalent fixierten Wirkstoffen und photospaltbaren Linkern
   Im Folgenden ist der prinzipielle Lösungsweg für den Einbau eines photochemisch aktivierbaren, epitheltoxischen Wirkstoffs in die Intraokularlinse bzw. die Zusatzausrüstung schematisch angegeben und exemplarisch erläutert.
   - Schritt 1: Herstellung funktionalisierter Monomerer, z.B. Cumarin-modifizierte Methacrylate oder Silikone
   - Schritt 2: Herstellung funktionalisierter Wirkstoffe, z.B. Cumarin-modifiziertes 5-Fluoruracil oder Mitomycin C
   - Schritt 3: Kopplung des funktionalisierten Monomers mit dem funktionalisierten Wirkstoff und gegebenenfalls Mischung mit weiterem Monomer.
   - Schritt 4: Mischung mit weiterem Polymer ohne gebundenem Wirkstoff
   - Schritt 5: Vernetzende Copolymerisation
   - Schritt 6: Formgebung

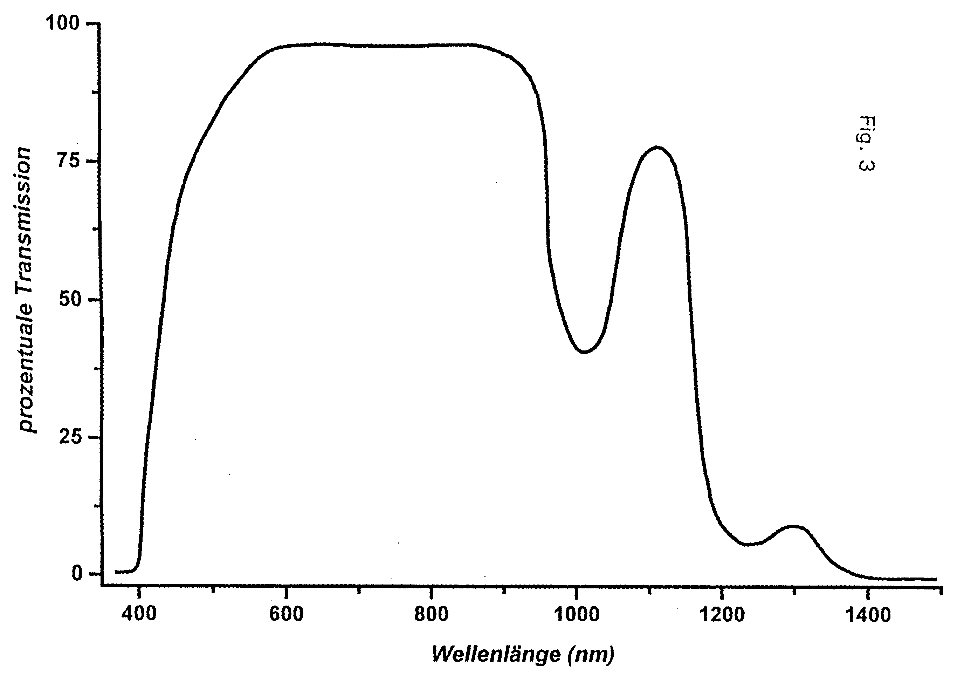

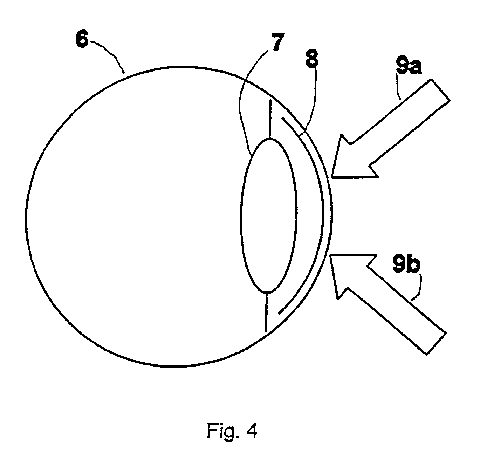

   Schritt 2 kann übersprungen werden, wenn der Wirkstoff selbst als Linker eingesetzt wird, z.B. Cumarin und seine Derivate.
   Nach Schritt 2 kann eine Polymerisation eingeschoben werden, um aus den Wirkstoff-Linker-Konjugaten Oligomere herzustellen. Die Schritte 3 und Schritt 5 können ggf. auch in umgekehrter Reihenfolge ausgeführt werden.
   Schritt 4 kann entfallen, wenn kein Polymerblend zur Einstellung der mechanischen Eigenschaften der Intraokularlinse nötig ist oder sonstwie die Verarbeitbarkeit des polymeren Werkstoffs beeinflusst werden soll.
   Schritt 5 kann entfallen bei Polymeren mit einer Glasübergangstemperatur (T_{g}) > 50°C.
   Schritt 5 und Schritt 6 können auch in einem Arbeitsgang zusammengefasst sein.
   Schritt 6 kann als vernetzende Copolymerisation durchgeführt werden. Dann wird die Formgebung vorzugsweise während der Polymerisation durchgeführt.
2.1 Grundmaterialien
   Als Ausgangsmaterial zur Herstellung der Intraokularlinse bzw. der Zusatzausrüstung wird auf die bekannten Materialien Polymethylmethacrylat (PMMA) und Silikon zurückgegriffen, die bereits zur Herstellung von kommerziell verfügbaren Intraokularlinsen eingesetzt werden. Silikonlinsen wurden in den letzten Jahren zunehmend eingesetzt, da sie wegen ihrer Faltbarkeit die Möglichkeit zur mikrochirurgischen Implantation bieten.
2.2 Wirkstoffe
   Als Wirkstoffe kommen alle bekannten cytotoxischen oder antineoplastischen Wirkstoffe in Betracht. Bevorzugt werden die in der Augenheilkunde etablierten cytotoxischen Verbindungen 5-Fluoruracil und Mitomycin C eingesetzt, die ein niedriges Molekulargewicht haben, was eine schnelle Diffusion (Minuten) aus der Intraokularlinse in das Kammerwasser gewährleistet. Weiter kommen Eflorinithine, Melphalcen, Daunorubicin, Cumarin und ähnliche Wirkstoffe in Betracht. Diese Verbindungen erfüllen die chemischen Voraussetzungen für eine kovalente Fixierung in besonders einfacher Form.
2.3 Photoaktivierbare Linker
   Die photochemisch induzierbare Abspaltung des Wirkstoffs aus dem Polymer mittels der Linkermoleküle ist in Fig. 2 verdeutlicht. Das linke Teilbild bezieht sich auf den Zustand vor der photochemischen Aktivierung, in dem der Wirkstoff über das Linkermolekül kovalent gebunden ist, während das rechte Teilbild den Zustand nach der photochemischen Aktivierung zeigt, in dem der Wirkstoff zusammen mit einem Linkermolekülfragment abgespalten und freigesetzt ist.
   Als photospaltbare Linkermoleküle werden bevorzugt Zimtsäurederivate wegen ihrer guten synthetischen Eigenschaften und Cumarin-Derivate wegen ihrer photoreversiblen Dimerisierung eingesetzt.
   Es ist bekannt, dass Cumarine im Zuge einer photochemisch induzierten [2 + 2] Cycloaddition dimerisieren (in der Regel λₘₐₓ > 300 nm). An niedermolekularen und polymergebundenen Cumarinen konnte gezeigt werden, dass die photochemisch induzierte Dimerisierung photochemisch reversibel ist (in der Regel *λ*ₘₐₓ < 300 nm). Diese reversible photochemische Reaktion bietet den Vorteil, dass die Abgabe cytotoxischer Substanzen mit Licht induziert und dosiert, aber auch im Rahmen der Herstellung zur Beladung mit Wirkstoff verwendet werden kann. Die Abgabe unerwünschter Nebenprodukte bei der Photolyse ist bisher nicht bekannt. Ähnliche Möglichkeiten bieten Zimtsäurederivate, deren Photodimerisierung bekannt ist.
3. Polymersynthesen
   Synthese der wirkstoffbeladenen Polymere
3.1 Kreuzdimerisation von Cumarinen
   Durch photochemische Kreuzdimerisation (vergl. Lit.: Y. Chen., C.-F. Chou, J. Polym. Sci., Polym. Chem. 33, 2705 (1995)) von OH- bzw. NH₂-funktionalisierten Cumarinderivaten mit unsubstituiertem Cumarin werden entsprechende Kreuzdimere (I) hergestellt. Deren Reinigung erfolgt durch Umkristallisation bzw. präparative Hochdruckchromatographie (HPLC). 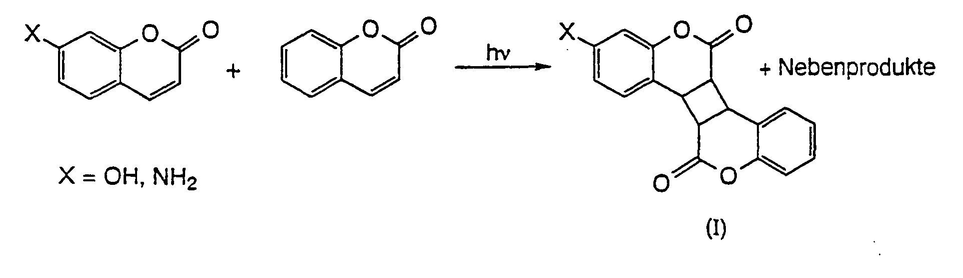
   Die Kreuzdimeren (I) werden in einem weiterem Reaktionsschritt mit ω-ungesättigten Fettsäuren, Acrylatderivaten (z.B. Acrylsäurechlorid) oder Methacrylatderivaten (z.B. Methacrylsäurechlorid) zu entsprechenden Estern bzw. Amiden umgesetzt (II). 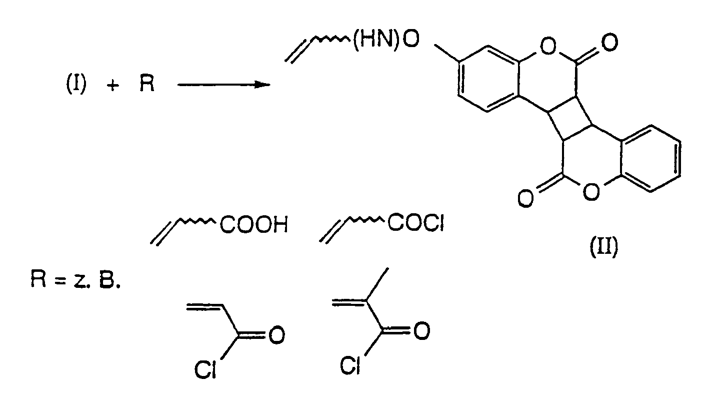
   Diese vinylfunktionalisierten Cumarinkreuzdimere (II) werden nun durch polymeranaloge Umsetzung mit Si-H-modifizierten Polydimethylsiloxanen an Polysiloxane gebunden bzw. durch Copolymerisation mit Olefinen (z. B. radikalische Copolymerisation mit Methylmethacrylat) polymergebunden.
3.2 Umsetzung mit Anhydriden
   OH- bzw. NH₂-funktionalisierte Cumarinderivate werden zunächst mit Dicarbonsäureanhydriden umgesetzt und silyliert. Anschließend werden NH₂-haltige Cytotoxica (z.B. Eflornithine) durch Amidierung mit der freien silylierten Carbonsäurefunktion des Cumarinderivates angebunden. 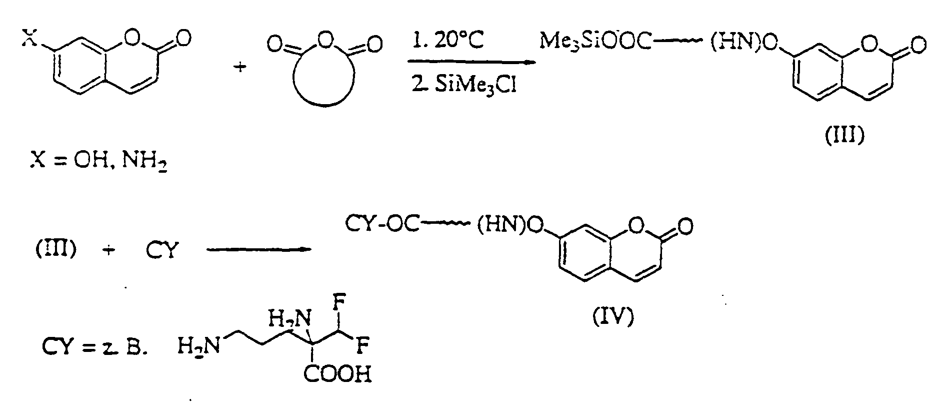
   Durch photochemische Kreuzdimerisation mit einem vinylfunktionalisierten Cumarinderivat und anschließende polymeranaloge Umsetzung bzw. Copolymerisation (siehe unter 3.1) können wirkstoffbeladene Polydimethylsiloxane bzw. Polymethacrylate und Polyacrylate hergestellt werden. 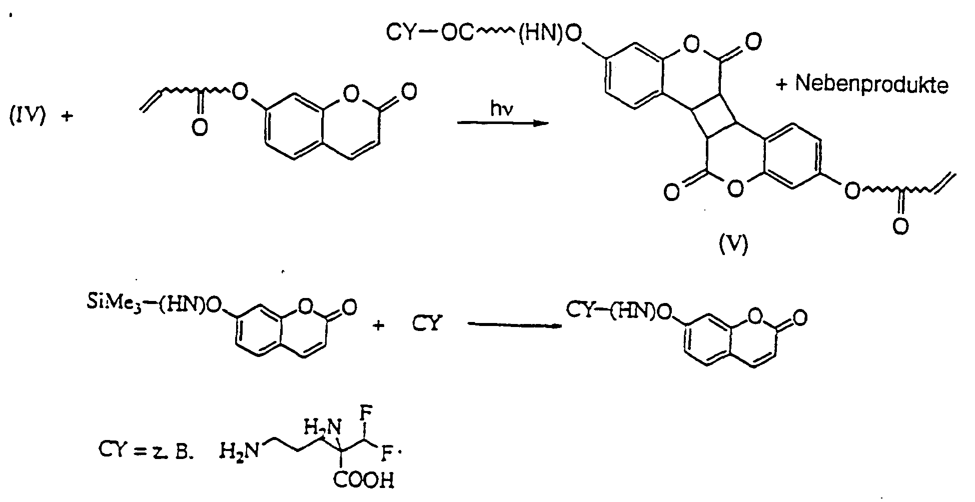
3.3 Umsetzung mit silylierten Cumarinderivaten
   Zunächst werden aminofunktionalisierte Cumarinderivate an der Aminfunktion silyliert und anschließend mit geeigneten Cytotoxica, z. B. Eflornithinen, durch eine Amidierungsreaktion verknüpft.
   Durch photochemische Kreuzdimerisation mit einem vinylfunktionalisierten Cumarinderivat (siehe 3.2) und anschließende polymeranaloge Umsetzung bzw. Copolymerisation (siehe unter 3.1) können wirkstoffbeladene Polydimethylsiloxane bzw. Polymethacrylate und Polyacrylate hergestellt werden.
4. Herstellung von Intraokularlinsen auf der Basis von polymeren Werkstoffen mit dispergierten Arzneistoffen
   Bei Verwendung von Polymethylmethacrylat (PMMA) als polymerem Werkstoff kann ein Wirkstoff-Precursor alternativ durch Eindiffundieren in den Werkstoff eingebracht werden. Zu diesem Zweck wird die PMMA-Probe mit einem Quellungsmittel aufgeweicht und mit einer den Wirkstoff-Precursor in gelöster Form enthaltenden Flüssigkeit in Kontakt gebracht. Dabei diffundiert der Wirkstoff-Precursor in die PMMA-Matrix ein. Nach Entfernen des Lösungsmittels erhält man eine Intraokularlinse mit eingebrachtem Depot an Wirkstoff-Precursor in makromolekularer Form, gelöst oder nanodispers verteilt.
   Aus dem Wirkstoff-Presursor kann durch photochemische Spaltung der Wirkstoff freigesetzt werden, der dann aus der Intraokularlinse ausdiffundieren kann.
5. Photochemische Freisetzung des Arzneistoffs durch Zweiphotonenabsorption
   In Fig. 4 ist schematisch ein Auge 6 mit UV-absorbierender Hornhaut 8 und implantierter Intraokularlinse 7 dargestellt. Die Intraokularlinse 7 enthält den freizusetzenden Wirkstoff. Zur Eliminierung von Linsenepithelzellen nach dem Abschluss der Wundheilung (Nachstar-Therapie) wird das Auge mit zwei Laserstrahlbündeln 9a und 9b unterschiedlicher Wellenlängen unter verschiedenen Winkeln bestrahlt. Die beiden die Hornhaut durchdringenden Laserstrahlbündel 9a und 9b werden mittels Linsen so fokussiert, dass sie sich in einem kleinen Volumen in oder auf der Wirkstoff-beladenen Intraokularlinse überlagern. Auf diese Weise wird im Schnittvolumen der Strahlenbündel eine photochemische Spaltung der Linkermoleküle durch Zweiphotonenabsorption induziert.
   Durch eine geeignete Kombination von Wellenlängen kann auf diese Weise eine räumlich scharf begrenzte photochemische Spaltung der Linkermoleküle im Überlappungsbereich der beiden Laserstrahlen ausgelöst werden.
   Für die Anwendung im Auge ist es natürlich nicht erwünscht, dass bereits Tageslicht zur Deliberation des Arzneistoffes führt. Die Hornhaut ist für Wellenlängen von weniger als 400 nm nur schlecht durchlässig. Eine Transmission von mehr als 90% wird zwischen 500 nm und 900 nm erzielt (vgl. z.B. W.J. Geeraets, E.R. Berry: Ocular spectral characteristics as related to hazards from lasers and other light sources. Am. J. Ophthalmol. 66 (1968) 15-20).
   Der Vorteil der Zweiphotonenaktivierung liegt darin, dass die photochemisch gewünschte Reaktion hinter der für die notwendige Primärwellenlänge absorptiven Hornhaut induziert werden kann. Ein Nachteil besteht allerdings darin, dass der Absorptionskoeffizient für die Zweiphotonenabsorption um Größenordnungen unter dem für die Einphotonenabsorption liegt. Es sind also am Ort der gewünschten Photochemie wesentlich höhere Energien notwendig als bei einer vergleichbaren Einphotonenreaktion. Dieses Problem kann jedoch mit Hilfe der beschriebenen optischen Anordnung gelöst werden, bei der mit fokussierten Laserstrahlen gearbeitet wird, die z.B. über Lichtleiter herangeführt und unter einem Winkel von beispielsweise 90° überlagert werden. Auf Grund der starken Fokussierung weiten sich die Strahlenbündel hinter dem Reaktionsort stark auf, so dass in der Ebene der Netzhautperipherie tolerable Intensitäten nicht überschritten werden.

## Patentansprüche

1. Ophthalmologisches Implantat (1, 2) zur Prophylaxe oder zur Behandlung von Kapseltrübung nach der Implantation einer synthetischen Linse,
**dadurch gekennzeichnet, dass** es aus einem polymeren Werkstoff besteht und zumindest bereichsweise eine chemische Komponente in immobilisierter Form umfasst, aus der durch photochemische Aktivierung ein pharmazeutischer Wirkstoff freisetzbar ist.

2. Ophthalmologisches Implantat nach Anspruch 1,
**dadurch gekennzeichnet, dass** es sich um eine Intraokularlinse (1) handelt.

3. Ophthalmologisches Implantat nach Anspruch 1,
**dadurch gekennzeichnet, dass** es sich um eine Zusatzausrüstung (2) für Intraokularlinsen handelt.

4. Ophthalmologisches Implantat nach einem der vorhergehenden Ansprüche, **dadurch gekennzeichnet, dass** der Wirkstoff eine zelltoxische Wirkung aufweist.

5. Ophthalmologisches Implantat nach einem der Ansprüche 1 bis 3,
**dadurch gekennzeichnet, dass** der Wirkstoff eine antibiotische, eine entzündungshemmende, eine antimikrobielle, eine antivirale oder/und eine fungizide Wirkung aufweist.

6. Ophthalmologisches Implantat nach einem der Ansprüche 1 bis 3,
**dadurch gekennzeichnet, dass** es sich bei dem Wirkstoff um ein Kortikosteroid, ein nicht-steroidales entzündungshemmendes Mittel oder einen Anti-Fibroplastenwachstumsfaktor handelt oder/und einen Wirkstoff, der die proliferative Vitreoretinopathie oder die Gewebefibrose hemmt.

7. Ophthalmologisches Implantat nach einem der vorhergehenden Ansprüche,
**dadurch gekennzeichnet, dass** der Wirkstoff kovalent an den polymeren Werkstoff gebunden ist.

8. Ophthalmologisches Implantat nach einem der vorhergehenden Ansprüche,
**dadurch gekennzeichnet, dass** der Wirkstoff an den polymeren Werkstoff über photochemisch spaltbare Linkermoleküle gekoppelt ist.

9. Ophthalmologisches Implantat nach Anspruch 8,
**dadurch gekennzeichnet, dass** die Linkermoleküle ausgewählt werden aus der Gruppe bestehend aus Zimtsäure, Cumarin und Derivaten davon.

10. Ophthalmologisches Implantat nach einem der Ansprüche 1 bis 6,
**dadurch gekennzeichnet, dass** der Wirkstoff oder/und eine Wirkstoff-Vorstufe in den polymeren Werkstoff eingebettet ist oder sind.

11. Ophthalmologisches Implantat nach einem der vorhergehenden Ansprüche,
**dadurch gekennzeichnet, dass** eine Wirkstoff-Vorstufe in feindisperser Form in den polymeren Werkstoff eingebettet ist.

12. Ophthalmologisches Implantat nach Anspruch 10,
**dadurch gekennzeichnet, dass** die mittlere Teilchengröße der dispergierten Wirkstoff-Vorstufe ≤ 1 µm beträgt.

13. Ophthalmologisches Implantat nach einem der vorhergehenden Ansprüche, **dadurch gekennzeichnet, dass** der polymere Werkstoff eine Wirkstoff-Vorstufe in oligomerer oder polymerer Form umfasst.

14. Ophthalmologisches Implantat nach einem der vorhergehenden Ansprüche,
**dadurch gekennzeichnet, dass** der polymere Werkstoff eine Wirkstoff-Vorstufe in Form von makromolekularen Konjugaten umfasst.

15. Ophthalmologisches Implantat nach einem der vorhergehenden Ansprüche,
**dadurch gekennzeichnet, dass** der Wirkstoff durch Einstrahlen von Licht in einem Spektralbereich freigesetzt wird, der nicht mehr als 50% des sichtbaren Spektralbereichs einnimmt.

16. Ophthalmologisches Implantat nach einem der vorhergehenden Ansprüche,
**dadurch gekennzeichnet, dass** die photochemische Aktivierung durch eine Zwei-Photonen-Absorption bewirkt wird.

17. Verfahren zur Herstellung eines ophthalmologischen Implantats, wie es in einem der Ansprüche 1 bis 16 definiert ist, umfassend die Schritte
- Bereitstellen eines polymeren Werkstoffes,
- Immobilisieren einer chemischen Komponente auf oder/und in den polymeren Werkstoff, aus der durch photochemische Aktivierung ein pharmazeutischer Wirkstoff freisetzbar ist, und
- Bilden eines ophthalmologischen Implantats aus dem polymeren Werkstoff.

18. Verfahren nach Anspruch 17 zur Herstellung eines ophthalmologischen Implantats, wie es in einem der Ansprüche 1 bis 16 definiert ist, **dadurch gekennzeichnet, dass** man einen polymeren Werkstoff bereitstellt, eine chemische Komponente auf oder/und in den polymeren Werkstoff immobilisiert, aus der durch photochemische Aktivierung ein pharmazeutischer Wirkstoff freisetzbar ist, und ein ophthalmologisches Implantat aus dem polymeren Werkstoff bildet.

19. Verfahren nach Anspruch 17 zur Herstellung eines ophthalmologischen Implantats, wie es in einem der Ansprüche 1 bis 16 definiert ist, **dadurch gekennzeichnet, dass** man einen polymeren Werkstoff bereitstellt, ein ophthalmologisches Implantat aus dem polymeren Werkstoff bildet und eine chemische Komponente auf oder/und in das Implantat immobilisiert, aus der durch photochemische Aktivierung ein pharmazeutischer Wirkstoff freisetzbar ist.

20. Verfahren nach einem der Ansprüche 17 bis 19, **dadurch gekennzeichnet, dass** eine chemische Komponente immobilisiert wird, aus der durch photochemische Aktivierung ein pharmazeutischer Wirkstoff freisetzbar ist, der eine zelltoxische Wirkung aufweist.

## Claims

1. Ophthalmologic implant (1, 2) for the prophylaxis or treatment of capsule opacification after implantation of a synthetic lens,
**characterized in that** the implant comprises a polymeric material and, at least in certain areas, a chemical component in an immobilized form, from which chemical component a pharmaceutically active substance will be released upon photochemical activation.

2. Ophthalmologic implant according to claim 1,
**characterized in that** the implant (1) is an intraocular lens.

3. Ophthalmologic implant according to claim 1,
**characterized in that** the implant is an auxiliary equipment (2) for intraocular lenses.

4. Ophthalmologic implant according to one of the preceding claims,
**characterized in that** the active substance has a cytotoxic effect.

5. Ophthalmologic implant according to claims 1 to 3,
**characterized in that** the active substance has an antibiotic, an anti-inflammatory, an antimicrobial, an antiviral or/and a fungicidal effect.

6. Ophthalmologic implant according to claims 1 to 3,
**characterized in that** the active substance is a corticosteroid, a non-steroidal anti-inflammatory agent or an anti-fibroblast growth factor or/and an active substance which inhibits proliferative vitreoretinopathy or tissue fibrosis.

7. Ophthalmologic implant according to one of the preceding claims,
**characterized in that** the active substance is covalently bound to the polymeric material.

8. Ophthalmologic implant according to one of the preceding claims,
**characterized in that** the active substance is coupled to the polymeric material via photochemically cleavable linker molecules.

9. Ophthalmologic implant according to claim 8,
**characterized in that** the linker molecules are selected from the group comprising cinnamic acid, coumarin and derivatives thereof.

10. Ophthalmologic implant according to one of claims 1 to 6,
**characterized in that** the active substance or/and a precursor of the active substance is or are embedded in the polymeric material.

11. Ophthalmologic implant according to one of the preceding claims,
**characterized in that** a precursor of the active substance is embedded in a finely dispersed form in the polymeric material.

12. Ophthalmologic implant according to claim 10,
**characterized in that** the average particle size of the dispersed precursor of the active substance is ≤ 1 µm.

13. Ophthalmologic implant according to one of the preceding claims,
**characterized in that** the polymeric material comprises a precursor of an active substance in an oligomeric or polymeric form.

14. Ophthalmologic implant according to one of the preceding claims,
**characterized in that** the polymeric material comprises a precursor of an active substance in the form of macromolecular conjugates.

15. Ophthalmologic implant according to one of the preceding claims,
**characterized in that** the active substance is released by irradiation with light in a spectral range which covers not more than 50 % of the visible spectral range.

16. Ophthalmologic implant according to one of the preceding claims,
**characterized in that** the photochemical activation is induced by a two-photon absorption.

17. Process for the production of an ophthalmologic implant as defined in one of claims 1 to 16, comprising the steps:
- providing a polymeric material,
- immobilizing a chemical component on or/and in the polymeric material, from which chemical component a pharmaceutically active substance can be released by photochemical activation, and
- forming an ophthalmologic implant from the polymeric material.

18. Process according to claim 17 for the production of an ophthalmologic implant as defined in one of claims 1 to 16, **characterized in that** a polymeric material is provided, a chemical component is immobilized on or/and in the polymeric material, from which chemical component a pharmaceutically active substance can be released by photochemical activation, and an ophthalmologic implant is formed from the polymeric material.

19. Process according to claim 17 for the production of an ophthalmologic implant as defined in one of claims 1 to 16, **characterized in that** a polymeric material is provided, an ophthalmologic implant is formed from the polymeric material and a chemical component is immobilized on or/and in the implant, from which chemical component a pharmaceutically active substance can be released by photochemical activation.

20. Process according to one of claims 17 to 19, **characterized in that** a chemical component is immobilized, from which chemical component a pharmaceutically active substance can be released by photochemical activation, which has a cytotoxic effect.

## Revendications

1. Implant ophtalmologique (1, 2) pour la prophylaxie ou le traitement de l'opacification capsulaire après implantation d'un cristallin synthétique,
**caractérisé en ce qu'**il est constitué du matériau polymère et comprend au moins dans certaines zones, un composant chimique sous forme immobilisée à partir duquel un principe actif pharmaceutique peut être libéré par activation photochimique.

2. Implant ophtalmologique selon la revendication 1, **caractérisé en ce qu'**il s'agit d'une lentille intra-oculaire (1).

3. Implant ophtalmologique selon la revendication 1, **caractérisé en ce qu'**il s'agit d'un équipement complémentaire (2) de lentilles intra-oculaires.

4. Implant ophtalmologique selon l'une quelconque des revendications précédentes, **caractérisé en ce que** le principe actif présente une action cytotoxique.

5. Implant ophtalmologique selon l'une quelconque des revendications 1 à 3, **caractérisé en ce que** le principe actif présente une action antibiotique, anti-inflammatoire, anti-microbienne, anti-virale et/ou fongicide.

6. Implant ophtalmologique selon l'une quelconque des revendications 1 à 3, **caractérisé en ce que** le principe actif est un corticostéroïde, un anti-inflammatoire non stéroïdien ou un facteur de croissance anti-fibroblastes et/ou un principe actif qui inhibe la vitréorétinopathie proliférante ou la fibrose tissulaire.

7. Implant ophtalmologique selon l'une quelconque des revendications précédentes, **caractérisé en ce que** le principe actif est lié de manière covalente au matériau polymère.

8. Implant ophtalmologique selon l'une quelconque des revendications précédentes, **caractérisé en ce que** le principe actif est couplé au matériau polymère par une molécule de liaison décomposable de manière photochimique.

9. Implant ophtalmologique selon la revendication 8, **caractérisé en ce que** la molécule de liaison est choisie dans le groupe comprenant l'acide cinnamique, la coumarine ou leurs dérivés.

10. Implant ophtalmologique selon l'une quelconque des revendications 1 à 6, **caractérisé en ce que** le principe actif et/ou un précurseur du principe actif est/sont intégré(s) dans le matériau polymère.

11. Implant ophtalmologique selon l'une quelconque des revendications précédentes, **caractérisé en ce qu'**un précurseur du principe actif est intégré sous forme finement dispersée dans le matériau polymère.

12. Implant ophtalmologique selon la revendication 10, **caractérisé en ce que** la taille moyenne de particules du précurseur de principe actif dispersé est ≤ 1 µm.

13. Implant ophtalmologique selon l'une quelconque des revendications précédentes, **caractérisé en ce que** le matériau polymère comprend un précurseur de principe actif sous une forme oligomère ou polymère.

14. Implant ophtalmologique selon l'une quelconque des revendications précédentes, **caractérisé en ce que** le matériau polymère comprend un précurseur de principe actif sous la forme de conjugués macromoléculaires.

15. Implant ophtalmologique selon l'une quelconque des revendications précédentes, **caractérisé en ce que** le principe actif est libéré par rayonnement de la lumière dans un domaine spectral qui n'occupe pas plus de 50 % du domaine spectral visible.

16. Implant ophtalmologique selon l'une quelconque des revendications précédentes, **caractérisé en ce que** l'activation photochimique s'effectue par une absorption à deux photons.

17. Procédé de fabrication d'un implant ophtalmologique tel que défini dans les revendications 1 à 16, comprenant les étapes consistant à
- proposer un matériau polymère,
- immobiliser un composant chimique sur et/ou dans le matériau polymère à partir duquel le principe actif pharmaceutique est libéré par activation photochimique, et
- former un implant ophtalmologique constitué du matériau polymère.

18. Procédé selon la revendication 17, pour la fabrication d'un implant ophtalmologique tel que défini dans les revendications 1 à 16, **caractérisé en ce que** l'on propose un matériau polymère, on immobilise un composant chimique sur et/ou dans le matériau polymère à partir duquel un principe actif pharmaceutique peut être libéré par activation photochimique et on forme un implant ophtalmologique constitué du matériau polymère.

19. Procédé selon la revendication 17, pour la fabrication d'un implant ophtalmologique tel que défini dans les revendications 1 à 16, **caractérisé en ce que** l'on propose un matériau polymère, on forme un implant ophtalmologique constitué du matériau polymère et on immobilise un composant chimique sur et/ou dans l'implant à partir duquel un principe actif pharmaceutique peut être libéré par activation photochimique.

20. Procédé selon l'une quelconque des revendications 17 à 19, **caractérisé en ce qu'**un composant chimique est immobilisé, à partir duquel un principe actif pharmaceutique qui présente une action cytotoxique, peut être libéré par activation photochimique.
